# EUROPÄISCHE PATENTSCHRIFT

(11) **EP 2 263 516 B1**
(45) Veröffentlichungstag und Bekanntmachung des Hinweises auf die Patenterteilung: **07.10.2015**
(21) Anmeldenummer: 10006013.6
(22) Anmeldetag: 10.06.2010
(51) Int. Cl.: A61B 1/04

(54) **Verfahren und Vorrichtung zum Steuern einer mehrfarbigen Ausgabe eines Bilds eines medizinischen Objekts**
Method and device for controlling a multi-colour display of an image from a medical object
Procédé et dispositif de commande d'une sortie multicolore d'une image d'un objet médical

(30) Priorität: 17.06.2009 DE 102009025662
(43) Veröffentlichungstag der Anmeldung: 22.12.2010
(73) Patentinhaber: Karl Storz GmbH & Co. KG, 78532 Tuttlingen (DE)
(72) Erfinder: Stepp, Herbert, Dr., 82152 Planegg (DE); Schachenmayr, Hilmar, 6030 Clarkson WA (AU)

(56) Entgegenhaltungen:
- EP-A1- 1 535 568
- WO-A1-2009/117483
- US-A1- 2005 065 406
- US-A1- 2008 239 070
- US-A1- 2009 041 368

## Beschreibung

Die vorliegende Erfindung bezieht sich auf ein Verfahren und eine Vorrichtung zum Steuern einer mehrfarbigen Ausgabe eines Bilds eines medizinischen Objekts.

Bei einigen bildgebenden diagnostischen Verfahren werden gleichzeitig oder nacheinander ein Bild eines mit Weißlicht beleuchteten medizinischen Objekts und ein Bild des medizinischen Objekts in Fluoreszenzlicht erfasst. Zu diesen Verfahren zählen die photodynamische Diagnose (PDD) und die Fluoreszenzendoskopie mit Indocyaningrün (ICG). Herkömmlich werden dafür zwei Kameras verwendet. Eine erste Kamera erfasst das aus der Beleuchtung des medizinischen Objekts mit Weißlicht resultierende Bild im sichtbaren Spektralbereich, eine zweite Kamera erfasst das Fluoreszenzlicht. Beide Kameras können beispielsweise über dichroide Spiegel mit einem einzigen Endoskop oder einem einzigen Objektiv gekoppelt sein, um beide Bilder aus der gleichen Perspektive zu erfassen.

Durch Verwendung zweier Kameras wird der apparative Aufwand verdoppelt. Insbesondere sind zwei Framegrabber bzw. Videograbber bzw. Bildfangschaltungen zur Digitalisierung der Videosignale der beiden Kameras erforderlich. Zur Echtzeitverarbeitung der beiden resultierenden Videostreams sind bei den Rechenkapazitäten der heute in der Medizintechnik verwendeten Computer zwei separate Systeme erforderlich. Auch das zu speichernde Datenvolumen ist verdoppelt und stellt entsprechende Anforderungen an Datenraten und Speicherkapazitäten.

Wenn nur ein einziges Kamerasystem sowohl für die Erfassung des Bilds in Weißlicht als auch des Bilds in Fluoreszenzlicht verwendet wird, können beide Bilder nur abwechselnd erfasst werden. Beim Umschalten zwischen beiden Modi müssen zumindest Beobachtungsfilter gewechselt werden. Beispielsweise ist bei Beobachtung des Fluoreszenzlichts in der Regel ein Filter erforderlich, das die remittierten Anteile des zur Anregung der Fluoreszenz verwendeten Lichts (im Folgenden: Anregungslicht) blockt. Selbst in Fällen, in denen eine nicht-gleichzeitige alternierende Erfassung eines Bilds in Weißlicht und eines Bilds in Fluoreszenzlicht hinnehmbar ist, stellt oft das Erfordernis eines Wechsels des Beobachtungsfilters ein unüberwindbares technologisches Hindernis dar. Beispielsweise ist es bislang nicht möglich, am distalen Ende eines Videoendoskops eine Vorrichtung zum Wechsel des Beobachtungsfilters zu integrieren.

Im Dokument US 2008(0239070 A1 ist ein Abbildungssystem mit einem einzigen Farbbildsensor zur simultanen Fluoreszenz- und Farbbild-Videoendoskopie beschrieben. Die zu beobachtende Probe wird kontinuier-lich mit Fluoreszenzanregungslicht und periodisch mit Beleuchtungslicht beleuchtet, wobei abwechselnd reine Fluoreszenz-Bilder und Farb-Plus-Fluoreszenz-Bilder erfasst werden, die voneinander subtrahiert werden, um reine Farbbilder zu erhalten. Ferner sind die Überlagerung von Fluoreszenz- und Färb- bzw. Weißlicht-Bildern, ein Weißabgleich und andere Korrekturen beschrieben.

Im Dokument WO 2009/117483 A1 ist ein Abbildungssystem zur kombinierten Abbildung in reflektiertem Vollfarblicht und in Licht im nahen Infrarot beschrieben. Ein zu beobachtender Bereich wird kontinuierlich mit blauem, grünem, rotem und infrarotem Licht beleuchtet, wobei zumindest entweder das rote Licht oder das infrarote Licht periodisch an- und ausgeschaltet werden. Synchronisiert mit der alternierenden Beleuchtung mit rotem und infrarotem Licht werden Bilder erfasst. Fehlende Bilddaten zu einem Farbkanal werden durch räumliche und/oder zeitliche Interpolation aus Bilddaten des gleichen Farbkanals erzeugt.

Das Dokument US 2009/0041368 A1 ist auf die Erzeugung von Luftbildern bezogen. Mit einer Mehrzahl von optischen Systemen werden jeweils Bilddaten zu unterschiedlichen Farbkanälen erfasst. Aus den Bilddaten wird ein Bild erzeugt. Die Anzahl der durch das erzeugte Bild dargestellten Farben ist größer als die Anzahl der optischen Systeme, von denen die Bilddaten er-fasst werden. Farben, die nicht von den optischen Systemen erfasst wer-den, werden unter Verwendung von Daten, die den Farben, die den unter-schiedlichen Farbkanälen entsprechen, zugeordneten sind, berechnet.

Eine Aufgabe der vorliegenden Erfindung besteht darin, ein verbessertes Verfahren und eine verbesserte Vorrichtung sowie ein entsprechendes Computer-Programm zum Steuern einer mehrfarbigen Ausgabe eines Bilds eines medizinischen Objekts zu schaffen.

Diese Aufgabe wird durch die Gegenstände der unabhängigen Ansprüche gelöst.

Weiterbildungen sind in den abhängigen Ansprüchen angegeben.

Verschiedene Ausführungsformen der vorliegenden Erfindung beruhen auf der Erkenntnis, dass gerade bei medizinischen Anwendungen nur ein Bruchteil des Farbraums ausgenutzt wird, und auf der Idee, einen der typischerweise drei oder vier Farbkanäle einer Kamera für die Erfassung von Fluoreszenzlicht zu verwenden und die Bildinformation dieses so für die Weißlichtdarstellung verlorenen Farbkanals aus den Bilddaten der verbleibenden Farbkanäle zu rekonstruieren. Aufgrund üblicher und statistisch ausgewerteter Korrelationen zwischen Farbwerten in relevanten Bildbereichen bzw. für relevante Objekte kann mit dem rekonstruierten Farbkanal bzw. den nachgebildeten Farbwerten zu dem für die Erfassung des Fluoreszenzlichts verwendeten Farbkanal eine oft sehr natürlich wirkende Farbgebung erzielt werden.

Medizinisches Personal muss bei diagnostischer oder therapeutischer, insbesondere chirurgischer, Tätigkeit sehr viel Information gleichzeitig aufnehmen und verarbeiten. Durch eine natürlich wirkende Farbgebung kann medizinisches Personal unterstützt und entlastet werden und ihm eine ermüdungsärmere Konzentration auf die eigentlich relevanten Informationen ermöglicht werden.

Dabei werden anstelle zweier Bildsensoren bzw. Kamerasysteme - je eines für die Erfassung eines Bilds in Weißlicht und für die Erfassung eines Bilds in Fluoreszenzlicht - nur ein (mehrfarbiger) Bildsensor bzw. ein Kamerasystem (ggf. mit einem dichroitisch wirkenden Prisma und mehreren an dem Prisma angeordneten Bildsensoren) verwendet. Zur Unterscheidung von Fluoreszenzlicht und remittiertem Weißlicht werden die Filterbeschichtungen des mehrfarbigen Bildsensors bzw. die dichroitischen Reflexionseigenschaften des Prismas einer Mehrchip-Kamera genutzt, insbesondere in Kombination mit einem angepassten Filterdesign. Dieses angepasste Filterdesign gewährleistet insbesondere, dass Fluoreszenzlicht nur von einem Farbkanal erfasst wird, während die verbleibenden Farbkanäle kein Fluoreszenzlicht sondern zumindest näherungsweise jeweils das Licht erfassen, das sie herkömmlich bei Beleuchtung mit Weißlicht erfassen würden.. Dabei wird in Kauf genommen, dass der zur Erfassung des Fluoreszenzlichts genutzte Farbkanal zur Erfassung des Bilds in Weißlicht verloren ist, die zugehörigen Farbwerte werden, wie erwähnt, rekonstruiert.

Abhängig von der Anwendung und der dazu gewählten Ausgestaltung bieten Ausführungsbeispiele der vorliegenden Erfindung eine Reihe von Vorteilen. Insbesondere kann in der Regel auf zwei separate Kamerasysteme für die Darstellung eines Weißlicht-Bilds und eines Fluoreszenzlicht-Bilds verzichtet werden. Der apparative Aufwand wird dadurch halbiert, insbesondere muss nur das Bild bzw. der Videostream einer einzigen Kamera erfasst, digitalisiert, verarbeitet und abgespeichert werden.

Ein weiterer Vorteil besteht oft darin, dass ein Wechsel von Beobachtungsfiltern nicht erforderlich ist. Die vorliegende Erfindung ist deshalb beispielsweise auch in Form eines Videoendoskops (d.h. mit Bildsensor am distalen Ende des Endoskops) implementierbar. Wenn kein Wechsel von Beobachtungsfiltern erforderlich ist, ist oft auch eine gleichzeitige Erfassung eines Bilds in Fluoreszenzlicht und eines (teilweise rekonstruierten) Bilds in Weißlicht möglich. Die vorliegende Erfindung ist sowohl mit einer Kamera mit einem einzigen mehrfarbigen Bildsensor als auch mit einer Mehrchip-Kamera mit dichroitischer Trennung der Farbkanäle nutzbar.

Bei einem Verfahren zum Steuern einer mehrfarbigen Ausgabe eines Bilds eines medizinischen Objekts zur Unterstützung von medizinischem Personal wird das medizinische Objekt mit Licht mit einem Beleuchtungsspektrum beleuchtet. Für eine Gruppe von einem oder mehreren Farbkanälen werden mittels eines Bildsensors Bilddaten, insbesondere Farbwerte, erfasst. In Abhängigkeit von den erfassten Bilddaten wird Bildinformation, insbesondere Farbwerte, betreffend einen weiteren Farbkanal, der nicht zu der Gruppe von einem oder mehreren Farbkanälen zählt, erzeugt, insbesondere berechnet. In Abhängigkeit von den erfassten Bilddaten und der erzeugten Bildinformation wird eine Bildausgabeeinrichtung zur mehrfarbigen Ausgabe des Bilds gesteuert.

Das Beleuchtungsspektrum ist beispielsweise ein weißes Spektrum oder ein vom menschlichen Auge als weiß wahrgenommenes Spektrum. Das Beleuchten des medizinischen Objekts und das Erfassen von Bilddaten können über eine oder zwei separate endoskopische Sonden erfolgen. Bei einem Bildsensor mit drei oder vier Farbkanälen umfasst die Gruppe zwei bzw. drei Farbkanäle.

Bildinformation betreffend den weiteren Farbkanal für einen Bildpunkt bzw. ein Pixel bzw. eine Bildzelle wird beispielsweise in Abhängigkeit von den erfassten Bilddaten für die Gruppe von einem oder mehreren Farbkanälen für diesen Bildpunkt und/oder in Abhängigkeit von erfassten Bilddaten für die Gruppe von einem oder mehreren Farbkanälen für umgebende Bildpunkte erzeugt. Bildinformation für einen Bildpunkt kann ferner in Abhängigkeit von zu verschiedenen Zeitpunkten erfassten Bilddaten erzeugt werden. Insbesondere wenn in Form einer Videoaufnahme nacheinander Bilddaten zu mehreren Bildem des medizinischen Objekts erfasst werden, kann das medizinische Objekt oder ein Teil des medizinischen Objekts in den mehreren Bildern zumindest entweder identifiziert oder verfolgt werden, um dem medizinischen Gegenstand bzw. dem Teil des medizinischen Objekts in jedem der mehreren Bilder Bildpunkte zuzuordnen. Bildinformation für einen Bildpunkt kann dann in Abhängigkeit davon erzeugt werden, ob der Bildpunkt dem medizinischen Objekt bzw. dem Teil des medizinischen Objekts zugeordnet ist. Auf diese Weise können zum Erzeugen von Bildinformation für verschiedene Objekte oder Teile von Objekten innerhalb des Bilds verschiedenen Funktionen oder Algorithmen oder Parameter verwendet werden.

Zum Erzeugen von Bildinformation kann insbesondere eine Lookup-Tabelle oder eine mathematische Funktion verwendet werden. Zur Erstellung der Lookup-Tabelle oder der mathematischen Funktion werden bei Beleuchtung eines Referenzobjekts mit Weißlicht oder Licht mit einem anderen vorbestimmten Spektrum Referenzbilddaten für die Gruppe von einem oder mehreren Farbkanälen und für den weiteren Farbkanal erfasst. Eine Korrelation zwischen Referenzbilddaten für den weiteren Farbkanal und Referenzbilddaten für die Gruppe von einem oder mehreren Farbkanälen wird ermittelt. Aufgrund der beispielsweise als Lookup-Tabelle oder in Form einer mathematischen Funktion abgelegten Korrelation kann Bildinformation betreffend den weiteren Farbkanal in Abhängigkeit von den erfassten Bilddaten für die Gruppe von einem oder mehreren Farbkanälen erzeugt werden.

Das Referenzobjekt kann ein hinsichtlich seiner Wechselwirkung mit Licht mit dem vorbestimmten Spektrum für abzubildende Objekte typisches Objekt sein. Die Korrelation kann auch anhand der Referenzbilddaten von mehreren Referenzobjekten ermittelt werden. Alternativ ist das Referenzobjekt das Objekt, wobei die Referenzbilddaten bei einer dafür vorgesehenen Beleuchtungssituation mit dem vorbestimmten Spektrum erfasst werden.

Bei einem der beschriebenen Verfahren kann das Objekt ferner gleichzeitig oder alternierend mit dem Beleuchten mit Licht mit dem Beleuchtungsspektrum mit Licht mit einem Anregungsspektrum, das von dem Beleuchtungsspektrum verschieden ist, beleuchtet werden. Mittels des Bildsensors oder eines weiteren Bildsensors können weitere Bilddaten für den weiteren Farbkanal erfasst werden, wobei insbesondere Fluoreszenzlicht von dem Objekt erfasst wird. Die Bildausgabeeinrichtung kann in Abhängigkeit von den erfassten weiteren Bilddaten oder in Abhängigkeit von den erfassten Bilddaten, der erzeugten Bildinformation und den erfassten weiteren Bilddaten gesteuert werden. Das Fluoreszenzlicht wird beispielsweise bei der Fluoreszenz von Indocyaningrün erzeugt.

Die beschriebenen Verfahren sind insbesondere mit nur einer Kamera ausführbar, wobei die Kamera einen Bildsensor für mehrere Farbkanäle oder mehrere Bildsensoren für je einen Farbkanal aufweist.

Die vorliegende Erfindung ist als Verfahren oder als Computer-Programm mit Programmcode zur Durchführung oder Steuerung eines solchen Verfahrens, wenn das Computer-Programm auf einem Computer oder einem Prozessor abläuft, implementierbar. Ferner ist die Erfindung als Computer-Programm-Produkt mit auf einem maschinenlesbaren Träger (beispielsweise einem ROM-, PROM-, EPROM-, EEPROM- oder Flash-Speicher, einer CD-ROM, DVD, HD-DVD, Blue-Ray-DVD, Diskette oder Festplatte) oder in Form von Firmware gespeichertem Programmcode zur Durchführung von einem der genannten Verfahren, wenn das Computer-Programm-Produkt auf einem Computer, Rechner oder Prozessor abläuft, implementierbar. Ferner kann die vorliegende Erfindung als digitales Speichermedium (beispielsweise ROM-, PROM-, EPROM-, EEPROM- oder Flash-Speicher, CD-ROM, DVD, HD-DVD, Blue-Ray-DVD, Diskette oder Festplatte) mit elektronisch auslesbaren Steuersignalen, die so mit einem programmierbaren Computer- oder Prozessor-System zusammenwirken können, dass eines der beschriebenen Verfahren ausgeführt wird, implementiert werden.

Ferner kann die vorliegende Erfindung als Vorrichtung implementiert werden, wobei die Vorrichtung ausgebildet ist, um eines der beschriebenen Verfahren auszuführen, oder wobei die Vorrichtung ein Computer-Programm, ein Computer-Programm-Produkt oder ein digitales Speichermedium umfasst, wie sie im vorangehenden Absatz beschrieben wurden.

Eine Vorrichtung zum Steuern einer mehrfarbigen Ausgabe eines Bilds eines medizinischen Objekts umfasst einen oder mehrere Bildsensoren zum Erfassen von Bilddaten, insbesondere Farbwerten, für eine Gruppe von einem oder mehreren Farbkanälen, eine Rekonstruktionseinrichtung und eine Steuerung. Die Rekonstruktionseinrichtung ist mit einem Ausgang des einen oder der mehreren Bildsensoren gekoppelt und ausgebildet, um Bildinformation, insbesondere Farbwerte, betreffend einen weiteren Farbkanal, der nicht zu der Gruppe von einem oder mehreren Farbkanälen zählt, in Abhängigkeit von den erfassten Bilddaten zu erzeugen. Die Steuerung ist mit dem Ausgang des einen oder der mehreren Bildsensoren und mit einem Ausgang der Rekonstruktionseinrichtung gekoppelt und ausgebildet, um eine Bildausgabeeinrichtung zur mehrfarbigen Ausgabe des Bilds in Abhängigkeit von den erfassten Bilddaten und der erzeugten Bildinformation zu steuern.

Die Vorrichtung kann ferner eine Beleuchtungseinrichtung zum alternierenden oder gleichzeitigen Beleuchten des medizinischen Objekts mit Licht mit einem Beleuchtungsspektrum und mit Licht mit einem Anregungsspektrum, das von dem Beleuchtungsspektrum verschieden ist, umfassen. Der eine oder die mehreren Bildsensoren sind dabei ferner zum Erfassen von Bilddaten für den weiteren Farbkanal ausgebildet. Die Steuerung ist dabei ferner zum Steuern der Bildausgabeeinrichtung in Abhängigkeit von den erfassten weiteren Bilddaten oder in Abhängigkeit von den erfassten Bilddaten, der erzeugten Bildinformation und den erfassten weiteren Bilddaten ausgebildet.

### Kurzbeschreibung der Figuren

Nachfolgend werden Ausführungsformen anhand der beigefügten Figuren näher erläutert. Es zeigen:
- Figur 1: eine schematische Darstellung eines medizinischen Objekts und einer Endoskopievorrichtung;
- Figur 2: eine schematische Darstellung einer Vorrichtung zum Steuern einer mehrfarbigen Ausgabe;
- Figur 3: eine schematische Darstellung von Emissions- und Transmissionsspektren;
- Figur 4: eine schematische Darstellung von Emissions-, Absorptions- und Transmissionsspektren;
- Figur 5: eine schematische Darstellung von Referenzbilddaten;
- Figur 6: ein schematisches Flussdiagramm eines Verfahrens zum Erzeugen einer Lookup-Tabelle;
- Figur 7: ein schematisches Flussdiagramm eines Verfahrens zum Steuern einer mehrfarbigen Ausgabe eines Bilds eines medizinischen Objekts.

### Beschreibung der Ausführungsformen

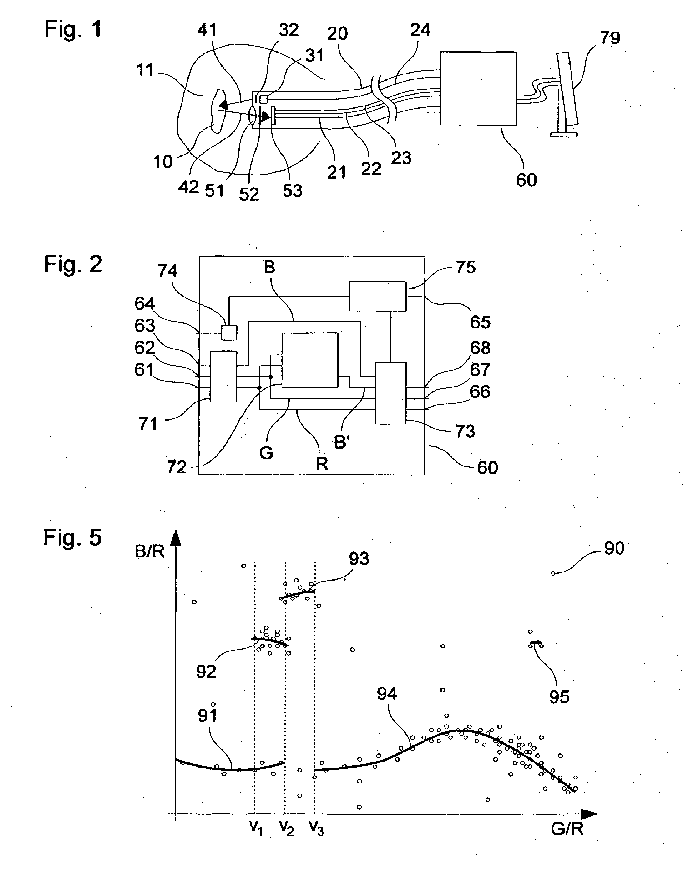

Figur 1 zeigt eine schematische Darstellung eines medizinischen Objekts 10 in einem Hohlraum 11, beispielsweise einer Mundhöhle eines Patienten, und einer endoskopischen Vorrichtung. Die endoskopische Vorrichtung umfasst ein Endoskop 20 mit Signalleitungen 21, 22, 23 und einer Beleuchtungsleitung 24. An einem distalen Ende des Endoskops 20 sind eine Lichtquelle 31 mit einem Beleuchtungsfilter 32, ein Objektiv 51, ein Beobachtungsfilter 52 und ein Bildsensor 53 angeordnet. Die Lichtquelle 31 ist mit der Beleuchtungsleitung 24 verbunden, um über diese elektrische Leistung zu erhalten. Der Bildsensor 53 ist mit den Signalleitungen 21, 22, 23 gekoppelt, deren Anzahl von drei abweichen kann.

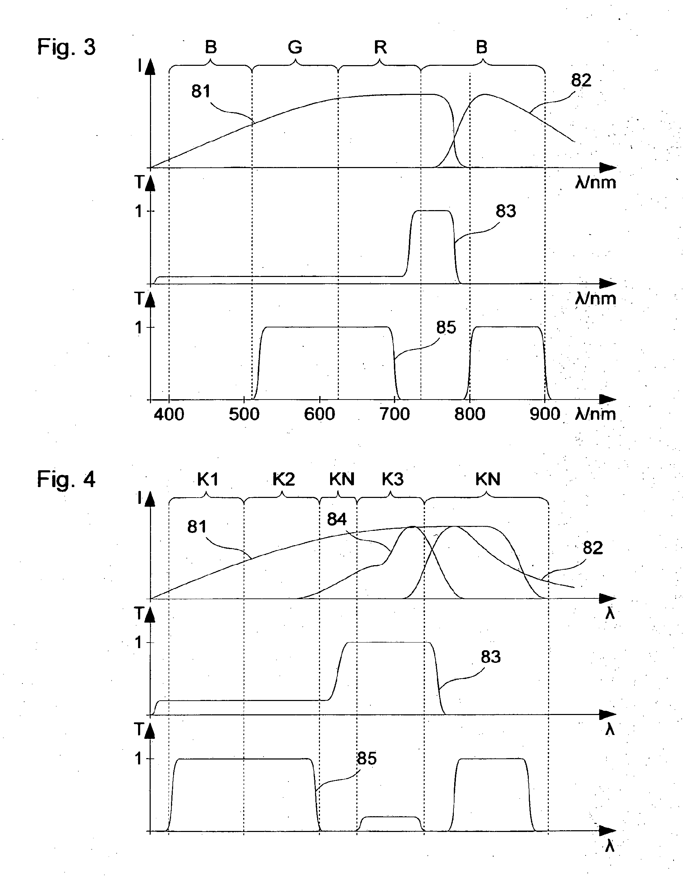

Ein proximales Ende des Endoskops 20 ist mit einer Vorrichtung 60 zum Steuern gekoppelt, die nachfolgend mit Bezug auf Figur 2 näher beschrieben wird. Die Vorrichtung 60 ist mit einer Bildausgabeeinrichtung 79, beispielsweise einem Bildschirm oder Monitor, gekoppelt. Die Lichtquelle 31 und das Beleuchtungsfilter 32 sind ausgebildet, um Licht 41 mit einem Beleuchtungsspektrum auf das medizinische Objekt 10 zu werfen. Das Licht 41 kann Anteile im ultravioletten, sichtbaren oder infraroten Spektralbereich haben. Das Objektiv 51 ist ausgebildet, um vom Objekt 10 remittiertes oder emittiertes Licht 42 auf den Bildsensor 53 zu fokussieren und so das medizinische Objekt 10 auf den Bildsensor 53 abzubilden. Eigenschaften des Beleuchtungsfilters 32 und des Beobachtungsfilters 52 werden nachfolgend mit Bezug auf die Figuren 3 und 4 näher beschrieben.

Die drei Signalleitungen 21, 22, 23 übertragen beispielsweise Signale, welche die Farbwerte zu jeweils einem von drei Farbkanälen des Bildsensors 53 darstellen. Beispielsweise überträgt die erste Signalleitung 21 das R-Signal, die zweite Signalleitung 22 das G-Signal und die dritte Signalleitung 23 das B-Signal des Bildsensors 53, wenn dieser ein RGB-Sensor ist. Diese Art der Signalisierung ermöglicht gerade in Bezug auf Figur 2 eine besonders anschauliche Darstellung. Tatsächlich ist die vorliegende Erfindung jedoch davon unabhängig, in welcher Weise Bilddaten vom Bildsensor 53 zur Vorrichtung 60 übertragen werden. Dies kann in komprimierter und unkomprimierter Form, elektrisch oder optisch und nach Farbkanälen getrennt oder mit Signalen, die mehrere oder alle Farbkanäle enthalten, erfolgen. Ferner ist die vorliegende Erfindung von dem verwendeten Farbraum unabhängig. Insbesondere sind anstelle des RGB-, HSV-, HSI-, HSL-, CMYK-, LMS-, XYZ-, Lab Farbraums auch beliebige andere Farbräume verwendbar. Die vom Bildsensor 53, bei der nachfolgend beschriebenen Rekonstruktion bzw. Aufbereitung und von der Bildausgabeeinrichtung 79 verwendeten Farbräume können gleich oder von einander verschieden sein. Allerdings ist eine Rekonstruktion bzw. Aufbereitung im originären Farbraum des Bildsensors 53 in der Regel vorteilhaft.

Einige Aspekte der vorliegenden Erfindung sind ferner unabhängig davon, ob der Bildsensor 53, wie in Figur 1 dargestellt, am distalen Ende des Endoskops 20 oder an seinem proximalen Ende oder in der Vorrichtung 60 angeordnet ist. Ferner kann die Lichtquelle 31 am distalen Ende des Endoskops 20 oder an seinem proximalen Ende oder in der Vorrichtung 60 angeordnet sein. In diesem Fall wird das Licht der Lichtquelle 31 beispielsweise über Lichtwellenleiter an das distale Ende des Endoskops 20 übertragen, wobei das Beleuchtungsfilter 32 nahe der Lichtquelle 31, am distalen Ende des Endoskops 20 oder dazwischen angeordnet sein kann. Abhängig davon, wo die Lichtquelle 31 angeordnet ist, kann sie beispielsweise eine oder mehrere Leuchtdioden, Laser, Xenen-Gasentladungslampen, Halogenmetalldampflampen, Quecksilberdampflampen, Halogenglühlampen umfassen. Anstelle des Beleuchtungsfilters 32 kann bereits die Lichtquelle 31 eine entsprechende spektrale Charakteristik aufweisen. Anstelle einer Lichtquelle 31 können mehrere Lichtquellen vorgesehen sein, die alternierend oder gleichzeitig verschiedene Spektren erzeugen. Das proximale Ende des Endoskops 20 kann, wie in Figur 1 dargestellt, unmittelbar oder aber über elektrische und/oder optische Leitungen mit der Vorrichtung 60 verbunden sein.

Figur 2 zeigt eine schematische Darstellung der Vorrichtung 60 zum Steuern einer mehrfarbigen Ausgabe des medizinischen Objekts 10. Die Vorrichtung 60 weist einen mit der ersten Signalleitung 21 gekoppelten ersten Signaleingang 61, einen mit der zweiten Signalleitung 22 gekoppelten zweiten Signaleingang 62, einen mit der dritten Signalleitung 23 gekoppelten dritten Signaleingang 63 und einen mit der Beleuchtungsleitung 24 gekoppelten Beleuchtungsausgang 64 auf. Ferner weist die Vorrichtung 60 einen Steuerungseingang 65 und drei Signalausgänge 66, 67, 68 zur Verbindung mit der Bildausgabeeinrichtung 79 auf. Der Steuerungseingang 65 ist beispielsweise zur Verbindung mit einer Tastatur, einer Maus oder anderen Bedieneinrichtungen einer Benutzerschnittstelle ausgebildet. Ähnlich wie die drei Signalleitungen 21, 22, 23 des Endoskops 20 und die drei Signaleingänge 61, 62, 63 der Vorrichtung 60 können auch die Signalausgänge 66, 67, 68 der Vorrichtung 60 jeweils einem von drei Farbkanälen R, G, B zugeordnet sein. Die Steuerung der Bildausgabeeinrichtung 79 kann jedoch auch in anderer Weise erfolgen.

Die Vorrichtung 60 umfasst ferner eine Signalaufbereitungseinrichtung 71, eine Rekonstruktionseinrichtung 72, eine Ausgabesteuerung 73, eine Beleuchtungssteuerung 74 und eine Modussteuerung 75. Die Signalaufbereitungseinrichtung 71 umfasst beispielsweise Eingangsverstärker, Dekodiereinrichtungen, Kodiereinrichtungen, Analog-Digital-Wandler und/oder Einrichtung zur Konvertierung von Bilddaten bzw. Farbwerten aus einem Farbraum in einen anderen Farbraum. Die Ausgabesteuerung 73 umfasst beispielsweise Einrichtungen zum Konvertieren von Farbwerten aus einem Farbraum in einen anderen Farbraum, Einrichtungen zum Mischen oder Ersetzen von Farben, Digital-Analog-Wandler und/oder Ausgangsverstärker. Die Beleuchtungssteuerung 74 ist zur Steuerung der Lichtquelle 31 oder ggf. mehrerer Lichtquellen ausgebildet. Die Modussteuerung 75 ist mit dem Steuerungseingang 65, der Ausgabesteuerung 73 und der Beleuchtungssteuerung 74 gekoppelt.

Ausgänge der Signalaufbereitungseinrichtung 71 sind mit Eingängen der Rekonstruktionseinrichtung 72 und der Ausgabesteuerung 73 gekoppelt. Einer oder mehrere Ausgänge der Rekonstruktionseinrichtung 72 ist/sind mit einem oder mehreren Eingängen der Ausgabesteuerung 73 gekoppelt.

Die nachfolgend beschriebene Rekonstruktion eines Farbkanals durch die Rekonstruktionseinrichtung 72 ist sowohl von den gewählten Farbräumen als auch von der Art der Signalisierung von Farbwerten außerhalb und innerhalb der Vorrichtung 60 unabhängig. Lediglich im Sinne einer anschaulichen Darstellung werden nachfolgend die Signalleitungen 21, 22, 23 des Endoskops 20, die Signaleingänge 61, 62, 63 und die Signalausgänge 66, 67, 68 der Vorrichtung 60 und die in Figur 2 innerhalb der Vorrichtung 60 dargestellten Leitungen zwischen der Signalaufbereitungseinrichtung 71, der Rekonstruktionseinrichtung 72 und der Ausgabesteuerung 73 jeweils einem Farbkanal R, G, B zugeordnet. Diese Zuordnung ist beispielhaft in Figur 2 angegeben, wobei der Farbkanal B' nachfolgend erklärt wird.

Im Folgenden wird beispielhaft davon ausgegangen, dass über die erste Signalleitung 21 des Endoskops 20 und den ersten Signaleingang 61 der Vorrichtung 60 das Signal R (Rotsignal), über die zweite Signalleitung 22 des Endoskops 20 und den zweiten Signaleingang 62 der Vorrichtung 60 das Signal G (Grünsignal) und über die dritte Signalleitung 23 des Endoskops 20 und den dritten Signaleingang 63 der Vorrichtung 60 das Signal B (Blausignal) übertragen wird. Ferner werde der Farbkanal B des Bildsensors 53 zur Erfassung von Fluoreszenzlicht genutzt, wodurch er der Erfassung eines Bilds aus Beleuchtung mit Weißlicht nicht mehr zur Verfügung steht.

Die Signale R und G werden nach Aufbereitung durch die Signalaufbereitungseinrichtung 71 der Ausgabesteuerung 73 und parallel dazu der Rekonstruktionseinrichtung 72 zugeleitet. Die Rekonstruktionseinrichtung 72 erzeugt in unten näher beschriebener Weise anhand der Signale R und G ein rekonstruiertes Signal B', das der Ausgabesteuerung 73 zugeleitet wird. Zusätzlich wird das von dem Bildsensor 53 erzeugte und von der Signalaufbereitungseinrichtung 71 aufbereitete Signal B der Ausgabesteuerung 73 zugeführt. Das Signal B enthält jedoch bei diesem Beispiel keine Information zu einer Abbildung des medizinischen Objekts 10 mittels Weißlicht, sondern Bilddaten zur Abbildung des medizinischen Objekts 10 in Fluoreszenzlicht.

Gesteuert durch die Modussteuerung 75 kann die Ausgabesteuerung 73 die Bildausgabeeinrichtung 79 so steuern, dass entweder nur ein rekonstruiertes Weißlicht-Bild oder nur ein Bild des medizinischen Objekts 10 in Fluoreszenzlicht oder gleichzeitig das rekonstruierte Weißlicht-Bild und das Bild in Fluoreszenzlicht durch die Bildausgabeeinrichtung 79 dargestellt werden. Das rekonstruierte Weißlicht-Bild wird dabei aus den vom Bildsensor 53 zu den Farbkanälen R und G erfassten Farbwerten bzw. Bilddaten und den Farbwerten zum rekonstruierten Farbkanal B' zusammengesetzt. Das Bild in Fluoreszenzlicht wird nur aus den Farbwerten zum vom Bildsensor 53 erfassten Farbkanal B gebildet.

Sowohl eine alleinige Darstellung eines Bilds in Fluoreszenzlicht als auch eine kombinierte Darstellung überlagerter Bilder in Weißlicht und in Fluoreszenzlicht ist mit einer Falschfarbendarstellung des Fluoreszenzlicht-Bilds möglich. Beispielsweise werden Bildpunkte, in denen die Intensität der Fluoreszenz eine vorbestimmte Schwelle überschreitet, in einer hoch oder maximal gesättigten Farbe, die bei dem medizinischen Objekt 10 sonst nicht auftritt, dargestellt.

Figur 3 zeigt eine schematische Darstellung von Emissions- und Transmissions-Spektren der Lichtquelle 31, einer Fluoreszenz des medizinischen Objekts 10, des Beleuchtungsfilters 32 und des Beobachtungsfilters 52. Den Abszissen ist die Wellenlänge □ in nm, den Ordinaten ist die Intensität I bzw. der Transmissionsgrad T zugeordnet. Aufgrund der relativen Ausrichtung der Abszissen ist nur die unterste Abszisse beschriftet.

Über dem obersten Diagramm in Figur 3 sind mit geschweiften Klammern näherungsweise die Wellenlängenbereiche angegeben, in denen die den Farbkanälen B, G, R zugeordneten Sensoren des Bildsensors 53 empfindlich sind. Das in Figur 3 dargestellte Beispiel beruht auf einem Bildsensor 53, dessen dem Farbkanal B zugeordnete Sensoren nicht nur im blauen Wellenlängenbereich in der Gegend von 400 nm bis 510 nm sondern auch in einem Wellenlängenbereich von unter 800 nm bis etwa 900 nm empfindlich sind.

Das oberste Diagramm zeigt die Intensitäten I in willkürlichen Einheiten für die Emission der Lichtquelle 31 und die Fluoreszenz des medizinischen Objekts 10 bzw. eines in diesem enthaltenen Fluoreszenzfarbstoffs. Da beide Intensitäten von geometrischen und anderen Faktoren abhängen, sind beide Spektren rein qualitativ. Das Spektrum 81 der Lichtquelle 31 umfasst Wellenlängen der Farbkanäle B, G und R zwischen ca. 400 nm und ca. 800 nm. Das Spektrum 82 der Fluoreszenz umfasst Wellenlängen von unter 800 nm bis weit über 900 nm mit einem Maximum bei ca. 820 nm. Es entspricht näherungsweise dem Fluoreszenzspektrum von Indocyaningrün (ICG). Damit sind die dem Farbkanal B zugeordneten Sensoren zur Erfassung der Fluoreszenz geeignet.

Das mittlere Diagramm in Figur 3 zeigt die Wellenlängenabhängigkeit des Transmissionsgrads 83 des Beleuchtungsfilters 32. Der Transmissionsgrad 83 des Beleuchtungsfilters 32 weist in großen Teilen der den Farbkanälen G und R zugeordneten Wellenlängenbereiche im Wesentlichen keine oder nur eine geringe Wellenlängenabhängigkeit auf. Licht in diesem Wellenlängenbereich wird hier auch als Beleuchtungslicht bezeichnet. Bei dem dargestellten Beispiel weist der Transmissionsgrad 83 des Beleuchtungsfilters 32 im blauen Bereich von ca. 400 nm bis ca. 500 nm eine ähnliche Größe auf. Abweichend davon kann der Transmissionsgrad 83 des Anregungsfilters 32 im blauen Bereich jedoch auch geringer oder Null oder näherungsweise Null sein.

In einem Bereich über 700 nm bis unter 800 nm ist der Transmissionsgrad 83 des Beleuchtungsfilters 32 wesentlich höher, insbesondere näherungsweise maximal (T ≈1 = 100%). Licht in diesem Wellenlängenbereich wird hier auch als Anregungslicht bezeichnet. Bei den für die Anregung des Fluoreszenzfarbstoffs relevanten Wellenlängen wird so eine wesentlich höhere Beleuchtungsintensität erzielt, um auch bei geringer Konzentration und/oder geringer Quantenausbeute des Fluoreszenzfarbstoffs eine hohe Intensität der Fluoreszenz zu erreichen, die zumindest näherungsweise der Intensität der Remission von grünem und rotem Licht entspricht.

Das unterste Diagramm in Figur 3 zeigt den Transmissionsgrad 85 des Beobachtungsfilters 52 in Abhängigkeit von der Wellenlänge. Der Transmissionsgrad 85 des Beobachtungsfilters 52 ist für die meisten Wellenlängen zwischen ca. 500 nm und ca. 700 nm und zwischen ca. 800 nm und ca. 900 nm maximal (T ≈1). In den Bereichen, in denen der Transmissionsgrad 83 des Beleuchtungsfilters 32 erhöht ist, ist der Transmissionsgrad 85 des Beobachtungsfilters 52 Null oder näherungsweise Null, um die vom medizinischen Objekt 10 remittierten Anteile des zur Anregung des Fluoreszenzfarbstoffs vorgesehenen Lichts zu blocken. Ferner ist der Transmissionsgrad 85 des Beobachtungsfilters 52 im blauen Bereich von ca. 400 nm bis ca. 500 nm Null oder im Wesentlichen Null, um zu vermeiden, dass die dem Farbkanal B zugeordneten Sensoren des Bildsensors 53 remittiertes blaues Licht empfangen.

Mit den gewählten Charakteristika des Beleuchtungsfilters 32 und des Beobachtungsfilters 52 erfassen die den Farbkanälen G und R zugeordneten Sensoren des Bildsensors 53 im Wesentlichen den grünen bzw. den roten Anteil des vom medizinischen Objekt 10 remittierten Beleuchtungslichts (auch als Weißlicht bezeichnet, das hier aber nur Wellenlängen bis ca. 700 nm umfasst), jedoch weder remittiertes Anregungslicht noch Fluoreszenzlicht. Die dem Farbkanal B zugeordneten Sensoren des Bildsensors 53 erfassen im Wesentlichen ausschließlich vom medizinischen Objekt 10 ausgehende bzw. von Fluoreszenzfarbstoff im medizinischen Objekt 10 ausgehende Fluoreszenz.

Damit wird vom medizinischen Objekt 10 remittiertes Licht mit den Wellenlängen des Farbkanals B nicht erfasst. Anders ausgedrückt ist der Farbkanal B zur Erfassung von Bilddaten, die einen ausgewogenen bzw. natürlichen Farbeindruck erzeugen, nicht verfügbar. Zur Erzielung eines ausgewogenen bzw. natürlichen Farbeindrucks werden Bildinformation bzw. Farbwerte zum Farbkanal B wie unten anhand der Figur 5 näher beschrieben rekonstruiert. Dies kann auch als Rekonstruktion eines Weißlichtbilds bezeichnet werden.

Figur 4 zeigt eine schematische Darstellung von Intensitäten I und Transmissionsgraden T bei einem weiteren Ausführungsbeispiel. Wiederum sind den Abszissen die Wellenlänge λ in nm und den Ordinaten die Intensität I bzw. der Transmissionsgrad T zugeordnet. Die Abszissen sind relativ zueinander ausgerichtet, jedoch nicht absolut beschriftet, da das anhand der Figur 4 dargestellte Beispiel unabhängig von konkreten Wellenlängen ist.

Über dem obersten Diagramm der Figur 4 sind mit geschweiften Klammern die Wellenlängenbereiche angedeutet, innerhalb derer die den Farbkanälen K1, K2, K3 und KN zugeordneten Sensoren des Bildsensors 53 empfindlich sind. Die nachfolgend beschriebenen Filtercharakteristika können entsprechend auf Situationen mit weiteren Farbkanälen K4, K5 etc. erweitert werden.

Im obersten Diagramm sind die Wellenlängenabhängigkeiten der Intensitäten I, der Emission der Lichtquelle 31, der Absorption des Fluoreszenzfarbstoffs im medizinischen Objekt 10 und der Fluoreszenz des Fluoreszenzfarbstoffs dargestellt. Das Spektrum 81 der Lichtquelle 31 umfasst im Wesentlichen alle den Farbkanälen K1, K2, K3, KN zugeordneten Wellenlängen. Das Absorptionsspektrum 84 umfasst Wellenlängen, die den Farbkanälen K3, KN zugeordnet sind, liegt aber überwiegend im Farbkanal K3. Das Spektrum 82 der Fluoreszenz umfasst im Wesentlichen Wellenlängen, die dem Farbkanal KN zugeordnet sind.

Im mittleren Diagramm ist die Wellenlängenabhängigkeit des Transmissionsgrads 83 des Beleuchtungsfilters 32 dargestellt. Der Transmissionsgrad 83 des Anregungsfilters 32 weist innerhalb der den Farbkanälen K1, K2 zugeordneten Wellenlängen einen ersten, niedrigen Wert auf. Bei den dem Farbkanal K3 zugeordneten Wellenlängen weist der Transmissionsgrad 83 einen zweiten, hohen Wert auf. Bei höheren Wellenlängen, die dem Farbkanal KN zugeordnet sind und die im Bereich des Spektrums 82 der Fluoreszenz des Fluoreszenzfarbstoffs liegen, ist der Transmissionsgrad 83 des Beleuchtungsfilters 32 Null oder im Wesentlichen Null.

Licht in den Wellenlängenbereichen der Farbkanäle K1 und K2 wird hier auch als Beleuchtungslicht bezeichnet. Licht in den Wellenlängenbereichen des Farbkanals K3 dient der Anregung des Fluoreszenzfarbstoffs und wird deshalb hier auch als Anregungslicht bezeichnet. Bei diesem Beispiel kann jedoch auch die Remission des Anregungslichts von dem medizinischen Objekt beobachtet werden.

Das unterste Diagramm zeigt den Transmissionsgrad 85 des Beobachtungsfilters 52. Dessen Transmissionsgrad 85 weist bei Wellenlängen, die den Farbkanälen K1, K2 und KN zugeordnet sind, einen hohen Wert T ≈1 auf. Bei Wellenlängen, die dem Farbkanal K3 zugeordnet sind, weist deren Transmissionsgrad 85 einen niedrigen Wert auf. Die Transmissionsgrade 83, 85 des Beleuchtungsfilters 32 und des Beobachtungsfilters 52 sind beispielsweise so gewählt, dass bei Wellenlängen, die den Farbkanälen K1, K2, K3 zugeordnet sind, das Produkt aus den Transmissionsgraden 83, 85 im Wesentlichen wellenlängenunabhängig ist. Der Transmissionsgrad 85 des Beobachtungsfilters 52 ist bei allen Wellenlängen, die dem Farbkanal KN zugeordnet sind und bei denen der Transmissionsgrad 83 des Beleuchtungsfilters 32 nicht im Wesentlichen Null ist, Null oder im Wesentlichen Null, um vom medizinischen Objekt 10 remittiertes Licht zu blocken.

Bei den anhand der Figur 4 dargestellten Filtercharakteristika empfangen die den Farbkanälen K1, K2, K3 zugeordneten Sensoren des Bildsensors 53 ausschließlich oder im Wesentlichen ausschließlich vom medizinischen Objekt 10 remittiertes Licht. Die dem Farbkanal KN zugeordnete Sensoren des Bildsensors 53 empfangen ausschließlich oder fast ausschließlich vom medizinischen Objekt 10 bzw. vom Fluoreszenzfarbstoff im medizinischen Objekt 10 ausgehendes Fluoreszenzlicht.

Damit wird vom medizinischen Objekt 10 remittiertes Licht mit den Wellenlängen des Farbkanals KN nicht erfasst. Anders ausgedrückt ist der Farbkanal KN zur Erfassung von Bilddaten, die einen ausgewogenen bzw. natürlichen Farbeindruck erzeugen, nicht verfügbar. Zur Erzielung eines ausgewogenen bzw. natürlichen Farbeindrucks werden Bildinformation bzw. Farbwerte zum Farbkanal KN wie nachfolgend anhand der Figur 5 näher beschrieben rekonstruiert. Dies kann auch als Rekonstruktion eines Weißlichtbilds bezeichnet werden.

Figur 5 zeigt eine schematische Darstellung von Referenzbilddaten. Dabei sind die Bilddaten zu einem Bildpunkt eines Referenzbilds jeweils durch einen kleinen Kreis 90 dargestellt. Der Abszisse ist das Verhältnis G/R zwischen dem Farbwert zum Farbkanal G und dem Farbwert zum Farbkanal R zugeordnet. Der Ordinate ist das Verhältnis B/R zwischen dem Farbwert zum Farbkanal B und dem Farbwert zum Farbkanal R zugeordnet. Die Anzahl der Bilddaten 90 ist gegenüber einem realen Bildsensor mit 10⁵ bis 10⁷ Bildpunkten bzw. Pixeln stark untertrieben. Aufgrund der Quantisierung der Farbwerte - üblicherweise werden ganzzeilige Werte zwischen Null und 255 verwendet - können mehrere Bilddaten an ein und demselben Punkt im Diagramm liegen.

Zur Gewinnung des in Figur 5 dargestellten Diagramms wird zunächst mit dem Bildsensor 53 ein Referenzbild eines Referenzobjekts erfasst. Dabei wird das Referenzobjekt mit Weißlicht oder Licht mit einem anderen vorbestimmten Beleuchtungsspektrum beleuchtet, bei dem ein vom Beobachter als natürlich bzw. ausgewogen empfundener Farbeindruck entsteht. In dem Referenzbild können relevante Bildbereiche ausgewählt, irrelevante Bildbereiche verworfen oder verschiedene Bildbereiche je nach Relevanz unterschiedlich gewichtet werden. Relevant sind Bildbereiche beispielsweise dann, wenn sie Gewebe darstellen oder in anderer Weise medizinisch relevant sind. Irrelevant sind Bildbereiche, die Instrumente, Zähne oder andere medizinisch für die vorgesehene Anwendung nicht relevante Gegenstände zeigen. Gegenständen, deren farbtreue Wiedergabe wichtig ist, wird eine hohe Relevanz zugeordnet, Gegenständen, deren farbtreue Wiedergabe weniger wichtig oder unwichtig ist, wird eine geringere Relevanz zugeordnet.

Alle Bilddaten zu Bildpunkten aus nicht verworfenen Bildbereichen werden in dem in Figur 5 dargestellten Diagramm dargestellt. Häufungen von Bilddaten werden identifiziert, Bilddaten in Bereichen des Diagramms mit einer geringen Dichte von Bilddaten werden ignoriert. Für die Häufungen wird eine Relation angepasst bzw. eine Funktion gefittet, beispielsweise wird ein Cubic-Spline berechnet. Dabei wird gegebenenfalls für jeden Bildpunkt die eingangs bestimmte Relevanz berücksichtigt. Der Relation kann abschnittsweise oder global angepasst werden. Weit ab einer Häufung liegende Bilddaten können vernachlässigt werden.

Die angepasste Relation kann so gewählt werden, dass sie für jeden Wert G/R eindeutig ist. Dies ist bei dem in Figur 5 gezeigten Beispiel unter anderem für einen ersten Abschnitt 91 für G/R < v₁ der Fall. Es können jedoch auch mehrere Häufungen von Bilddaten so ausgewertet werden, dass zu einem Wert von G/R mehrere angepasste Relationen existieren. Dies ist beispielsweise für den ersten Abschnitt 91 und einen zweiten Abschnitt 92 für v₁ < G/R < V₂, für den zweiten Abschnitt 92 und einen dritten Abschnitt 93 in einem kleinen Bereich um G/R = v₂ und für einen vierten Abschnitt 94 und einen fünften Abschnitt 95 in einem kleinen Intervall von G/R der Fall.

Die Abschnitte 91, 92, 93, 94, 95 der angepassten Relation werden in eine eindeutige oder mehrdeutige Lookup-Tabelle übertragen. Beim vorliegenden Beispiel müsste die Lookup-Tabelle im Bereich des zweiten Abschnitts 92 und im Bereich des fünften Abschnitts 95 mehrere Einträge aufweisen bzw. mehrdeutig sein. Anstelle einer Lookup-Tabelle können beispielsweise auch die Parameter der angepassten Relation abgelegt werden.

Eine entsprechende Auswertung von Bilddaten ist auch in anderen Farbräumen möglich, beispielsweise im HSV- oder CMYK-Farbraum. Die in Figur 5 dargestellte Normierung der Farbwerte zu den Farbkanälen G und B mit dem Farbwert zum Farbkanal R ist bei medizinischen Anwendungen vorteilhaft, da Rottöne dominieren und deshalb die Werte von G/R und B/R überwiegend kleiner als 1 sind. Damit stellt die Normierung mit dem Farbwert zum Farbkanal R näherungsweise eine Normierung auf die Helligkeit dar, die beispielsweise bei endoskopischen Anwendungen vom Abstand des Objekts vom distalen Ende des Endoskops abhängt. Bei Verwendung des HSV-Modells würden beispielsweise H und S gegeneinander aufgetragen. Bei insgesamt nur zwei Farbkanälen ist beispielsweise eine Normierung mit der Summe beider Farbwerte oder mit einer in einer vorbestimmten Umgebung um den betreffenden Bildpunkt gemittelten Summe beider Farbwerte möglich.

Die anhand der Figur 5 dargestellte Auswertung erfolgt beispielsweise mit Bezug auf eines oder mehrere Referenzbilder, die repräsentativ für bei einer vorbestimmten Anwendung erfasste Bilder sind, oder auch mit Bezug auf eines oder mehrere Referenzbilder, die zu Zeitpunkten erfasst wurden, zu denen keine Fluoreszenz angeregt, kein Fluoreszenzlicht emittiert sondern das Objekt mit Weißlicht beleuchtet und ohne Filter, das ein Weißlichtspektrum verändert, beobachtet wurde. Die letztgenannten Bedingungen werden auch als Weißlichtmodus bezeichnet. Dieser kann beispielsweise alternierend zu einem Fluoreszenzmodus gesteuert bzw. verwendet werden. Im Fluoreszenzmodus kann dann jeweils die beim letzten vorangehenden Weißlichtmodus erzeugte Lookup-Tabelle verwendet werden.

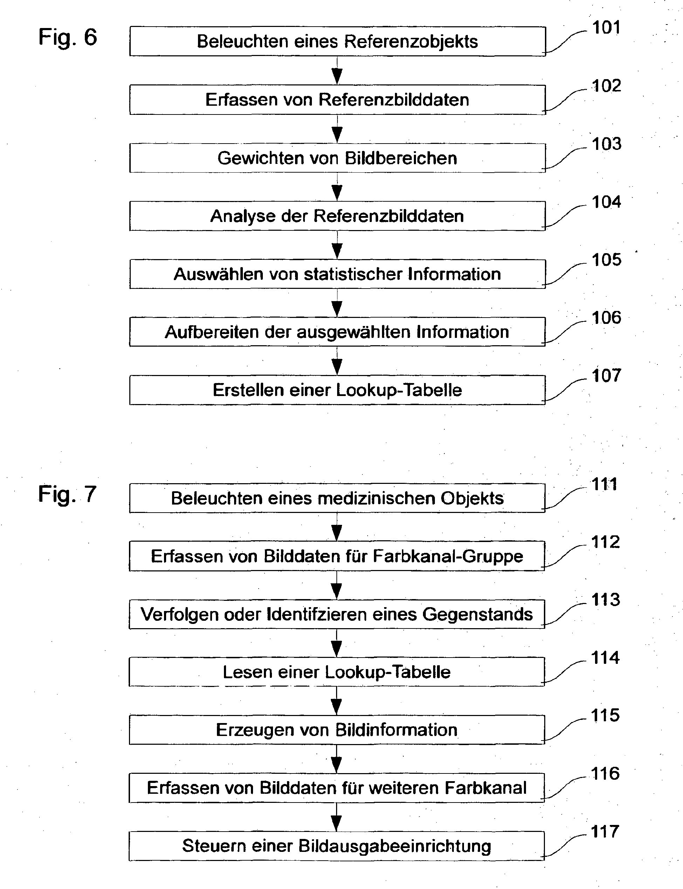

Die Figuren 6 und 7 zeigen schematische Flussdiagramme eines Verfahrens zum Erstellen einer Lookup-Tabelle bzw. eines Verfahrens zum Steuern einer mehrfarbigen Ausgabe eines Bilds. Beide Verfahren können im Betrieb einer Vorrichtung zum Steuern einer mehrfarbigen Ausgabe kombiniert werden, insbesondere unmittelbar nacheinander oder alternierend ausgeführt werden. Obwohl beide Verfahren auch mit Vorrichtungen, Filtercharakteristika und Farbräumen ausführbar sind, die sich von den oben anhand der Figuren 1 bis 5 dargestellten unterscheiden, werden nachfolgend Bezugszeichen aus den Figuren 1 bis 5 verwendet, um das Verständnis zu erleichtern.

Bei einem Verfahren zum Erstellen einer Lookup-Tabelle wird bei einem ersten Schritt 101 ein Referenzobjekt mit Weißlicht oder Licht mit einem anderen vorbestimmten Spektrum beleuchtet. Bei einem zweiten Schritt 102 werden Referenzbilddaten erfasst. Bei einem dritten Schritt 103 werden in dem Referenzbild Bildbereiche nach ihrer medizinischen Relevanz bzw. nach der Wichtigkeit ihrer farbtreuen bzw. natürlichen Darstellung gewichtet und/oder ausgewählt und/oder verworfen.

Bei einem vierten Schritt 104 werden die nicht verworfenen Referenzbilddaten analysiert. Beispielsweise werden Häufungen identifiziert, der Farbraum gewechselt oder mathematische Umformungen vorgenommen. Bei einem fünften Schritt 105 wird statistische Information aus den analysierten Referenzbilddaten ausgewählt. Dabei werden insbesondere solche Referenzbilddaten berücksichtigt, die vielen anderen Referenzbilddaten ähnlich sind. Dies ist bei der Darstellung in Figur 5 in den beschriebenen Häufungen gegeben. Dabei kann die Relevanz für jeden einzelnen Bildpunkt berücksichtigt werden.

Der erste Schritt 101, der zweite Schritt 102, der dritte Schritt 103, der vierte Schritt 104 und der fünfte Schritt 105 können für mehrere Referenzobjekte und mehrere Bilder von einem oder mehreren Referenzobjekten wiederholt werden.

Bei einem sechsten Schritt 106 wird die ausgewählte statistische Information aufbereitet, beispielsweise durch einen Cubic-Spline oder Anpassen einer anderen Relation. Dabei kann wiederum für jeden Bildpunkt seine Relevanz berücksichtigt werden. Bei einem siebten Schritt 107 wird aus der aufbereiteten ausgewählten Information eine Lookup-Tabelle erstellt. Diese Lookup-Tabelle enthält beispielsweise für jeden Wert von G/R einen oder mehrere Werte von B/R oder für jedes Wertepaar von R und G einen Wert von B. Anstelle einer Lookup-Tabelle kann eine mathematische Funktion oder Relation abgelegt werden. Die Lookup-Tabelle bzw. die mathematische Funktion oder Relation kann beim nachfolgend anhand der Figur 7 dargestellten Verfahren verwendet werden.

Bei manchen Anwendungen kann ein Verfahren, wie es oben anhand der Figur 6 dargestellt wurde, ganz oder teilweise periodisch oder aperiodisch wiederholt werden. Beispielsweise kann das Verfahren immer dann wiederholt werden, wenn das medizinische Objekt mit Weißlicht oder Licht mit einem anderen vorbestimmten Spektrum, das ohne Rekonstruktion eines Farbkanals einen natürlichen bzw. ausgewogenen Farbeindruck hervorruft, beleuchtet wird (nachfolgend auch als Weißlichtmodus bezeichnet). Eine weitere Voraussetzung ist, dass kein Beobachtungsfilter oder ein Beobachtungsfilter 52, der den Farbeindruck nicht verfälscht, insbesondere eine in allen Farbkanälen näherungsweise gleiche Transparenz aufweist, verwendet wird.

Figur 7 zeigt ein schematisches Flussdiagramm eines Verfahrens zum Steuern einer mehrfarbigen Ausgabe eines Bilds eines medizinischen Objekts. Bei einem ersten Schritt 111 wird das medizinische Objekt mit einem Beleuchtungsspektrum beleuchtet. Bei einem zweiten Schritt 112 werden Bilddaten, insbesondere Farbwerte, für eine Gruppe von einem oder mehreren Farbkanälen mittels eines Bildsensors 53 erfasst.

Bei einem optionalen dritten Schritt 113 wird der medizinische Gegenstand oder ein Teil des medizinischen Gegenstands innerhalb des Bilds identifiziert, beispielsweise unter Verwendung von Algorithmen, die als Objekterkennung bekannt sind. Alternativ oder zusätzlich kann ein Gegenstand über mehrere nacheinander erfasste Bilder verfolgt werden. Dem Gegenstand kann dann eine eigene Lookup-Tabelle oder ein eigener Teil einer Lookup-Tabelle zugeordnet sein, die beispielsweise bei einem zurückliegenden Weißlichtmodus wie oben anhand der Figur 6 beschrieben erstellt wurde.

Bei einem vierten Schritt 114 wird anhand der beim zweiten Schritt 112 erfassten Farbwerte für die Gruppe von einem oder mehreren Farbkanälen ein Farbwert für einen weiteren Farbkanal, für den beim zweiten Schritt 112 kein Farbwert erfasst wurde, gelesen. Dabei kann eine Lookup-Tabelle aus mehreren Lookup-Tabellen oder einer von mehreren Einträgen in einer Lookup-Tabelle aufgrund der beim dritten Schritt 113 festgestellten Identität eines Gegenstands, dem der betreffende Bildpunkt zugeordnet ist, ausgewählt werden. Bei einer Mehrdeutigkeit, wie sie oben anhand der Figur 5 beispielsweise für G/R = v₂ vorliegt, können Bilddaten bzw. Farbwerte zu umliegende Bilddaten berücksichtigt werden. Wenn für umliegende Bildpunkte meist G/R > v₂ gilt, wird für B/R der zum Abschnitt 93 korrespondierende Eintrag in der Lookup-Tabelle gelesen, wenn für umgebende Bildpunkte überwiegend G/R < v₂ gilt, wird aus der Lookup-Tabelle der zum zweiten Abschnitt 92 korrespondierende Eintrag gelesen. Für v₁ < G/R < v₂ wird beispielsweise anhand der Identität des Gegenstands, dem der Bildpunkt zuzuordnen ist, entschieden, ob der dem ersten Abschnitt 91 zugeordnete Eintrag oder dem zweiten Abschnitt 92 zugeordnete Eintrag zu lesen ist.

Bei einem fünften Schritt 115 wird anhand des beim vierten Schritt 114 aus der Lookup-Tabelle gelesenen Werts Bildinformation erzeugt, beispielsweise durch Berechnung eines Farbwerts zum Farbkanal B aus dem gelesenen Wert von B/R und dem Farbwert zum Farbkanal R. Bei einem sechsten Schritt 116 werden Bilddaten, insbesondere Farbwerte, für einen weiteren Farbkanal erfasst. Bei dem oben anhand der Figuren 2 und 3 dargestellten Beispiel würde der Farbwert für den Farbkanal B erfasst, dem Fluoreszenzlicht zugeordnet ist. Bei einem siebten Schritt 117 wird eine Bildausgabeeinrichtung 79 gesteuert, um entweder nur ein rekonstruiertes Weißlicht-Bild aus den beim zweiten Schritt 112 erfassten Farbwerten und dem beim fünften Schritt 115 rekonstruierten Farbwert oder nur ein Bild aus dem beim sechsten Schritt 116 erfassten Farbwert zur Fluoreszenz oder beides nebeneinander oder überlagert darzustellen.

Die dargestellten Verfahren und Vorrichtungen sind auch für medizinische Anwendungen, bei denen keine Fluoreszenz erfasst wird, und für nicht-medizinische Anwendungen einsetzbar, um bei Ausfall eines Farbkanals eine möglichst farbtreue Wiedergabe zu rekonstruieren.

Bezugszeichen
- 10: Medizinisches Objekt
- 11: Hohlraum
- 20: Endoskop
- 21: erste Signalleitung
- 22: zweite Signalleitung
- 23: dritte Signalleitung
- 24: Beleuchtungsleitung zur Lichtquelle 31
- 31: Lichtquelle
- 32: Beleuchtungsfilter
- 41: Licht mit Beleuchtungsspektrum
- 42: Licht vom Objekt 10
- 51: Objektiv
- 52: Beobachtungsfilter
- 53: Bildsensor
- 60: Vorrichtung zum Steuern
- 61: erster Signaleingang der Vorrichtung 60
- 62: zweiter Signaleingang der Vorrichtung 60
- 63: dritter Signaleingang der Vorrichtung 60
- 64: Beleuchtungsausgang der Vorrichtung 60
- 65: Steuerungseingang der Vorrichtung 60
- 66: erster Signalausgang der Vorrichtung 60
- 67: zweiter Signalausgang der Vorrichtung 60
- 68: dritter Signalausgang der Vorrichtung 60
- 71: Signalaufbereitungseinrichtung
- 72: Rekonstruktionseinrichtung
- 73: Ausgabesteuerung
- 74: Beleuchtungssteuerung
- 75: Modussteuerung
- 79: Bildausgabeeinrichtung
- 81: Spektrum der Lichtquelle 31
- 82: Spektrum der Fluoreszenz eines Fluoreszenzfarbstoffs
- 83: Transmissionsgrad des Beleuchtungsfilters 32
- 84: Absorptionsspektrum des Fluoreszenzfarbstoffs
- 85: Transmissionsgrad des Beobachtungsfilters 52
- 90: Bilddaten zu einem Bildpunkt eines Referenzbilds
- 91: erster Abschnitt einer angepassten Relation
- 92: zweiter Abschnitt einer angepassten Relation
- 93: dritter Abschnitt einer angepassten Relation
- 94: vierter Abschnitt einer angepassten Relation
- 95: fünfter Abschnitt einer angepassten Relation
- 101: erster Schritt
- 102: zweiter Schritt
- 103: dritter Schritt
- 104: vierter Schritt
- 105: fünfter Schritt
- 106: sechster Schritt
- 107: siebter Schritt
- 111: erster Schritt
- 112: zweiter Schritt
- 113: dritter Schritt
- 114: vierter Schritt
- 115: fünfter Schritt
- 116: sechster Schritt
- 117: siebter Schritt

## Patentansprüche

1. Verfahren zum Steuern einer mehrfarbigen Ausgabe eines Bilds eines medizinischen Objekts (10) zur Unterstützung von medizinischem Personal, mit folgenden Schritten:
Beleuchten (111) des medizinischen Objekts (10) mit Licht mit einem Beleuchtungsspektrum;
Erfassen (112) von Bilddaten für eine Gruppe von einem oder mehreren Farbkanälen mittels eines Bildsensors (53);
Erzeugern (115) von Bildinformation betreffend einen weiteren Farbkanal, der nicht zu der Gruppe von einem oder mehreren Farbkanälen zählt, in Abhängigkeit von den erfassten Bilddaten, wobei in die Erzeugung der Bildinformation keine Bilddaten betreffend den weiteren Farbkanal eingehen;
Steuern (117) einer Bildausgabeeinrichtung (79) zur mehrfarbigen Ausgabe des Bilds in Abhängigkeit von den erfassten Bilddaten und der erzeugten Bildinformation;
Beleuchten (111) des medizinischen Objekts (10) mit Licht mit einem Anregungsspektrum, das von dem Beleuchtungsspektrum verschieden ist;
Erfassen (116) von weiteren Bilddaten für den weiteren Farbkanal mittels des Bildsensors (53) oder eines weiteren Bildsensors,
wobei die Bildausgabeeinrichtung (79) in Abhängigkeit von den erfassten weiteren Bilddaten oder in Abhängigkeit von den erfassten Bilddaten, der erzeugten Bildinformation und den erfassen weiteren Bilddaten gesteuert wird,
wobei das medizinische Objekt (10) aufgrund der Beleuchtung mit dem Anregungsspektrum Fluoreszenzlicht emittiert
und das Fluoreszenzlicht über den weiteren Farbkanal erfasst wird.

2. Verfahren nach dem vorangehenden Anspruch, bei dem das Beleuchten (111) und das Erfassen (112) über eine endoskopische Sonde (20) erfolgen.

3. Verfahren nach einem der vorangehenden Ansprüche, bei dem Bildinformation für einen Bildpunkt zumindest entweder in Abhängigkeit von den erfassten Bilddaten für diesen Bildpunkt oder in Abhängigkeit von erfassten Bilddaten für umgebende Bildpunkte erzeugt wird.

4. Verfahren nach einem der vorangehenden Ansprüche, bei dem Bildinformation für einen Bildpunkt in Abhängigkeit von zu verschiedenen Zeitpunkten erfassten Bilddaten erzeugt wird.

5. Verfahren nach dem vorangehenden Anspruch, bei dem
nacheinander Bilddaten zu mehreren Bildern des medizinischen Objekts (10) erfasst werden,
das medizinische Objekt (10) oder ein Teil des medizinischen Objekts (10) in den mehreren Bildern zumindest entweder identifiziert oder verfolgt wird, um dem medizinischen Objekt (10) bzw. dem Teil des medizinischen Objekts (10) Bildpunkte zuzuordnen,
Bildinformation für einen Bildpunkt in Abhängigkeit von der Zuordnung des Bildpunkts zu dem medizinischen Objekt (10) bzw. zu dem Teil des medizinischen Objekts (10) erzeugt wird.

6. Verfahren nach einem der vorangehenden Ansprüche, bei dem zum Erzeugen von Bildinformation eine Lookup-Tabelle verwendet wird.

7. Verfahren nach einem der vorangehenden Ansprüche, ferner mit folgendem Schritt:
Erfassen (102) von Referenzbilddaten für die Gruppe von einem oder mehreren Farbkanälen und für den weiteren Farbkanal bei Beleuchtung (101) eines Referenzobjekts mit Weißlicht oder Licht mit einem anderen vorbestimmten Spektrum;
Ermitteln (106) einer Korrelation zwischen Referenzbilddaten für den weiteren Farbkanal und Reterenzbilddaten für die Gruppe von einem oder mehreren Farbkanälen,
wobei die Bildinformation betreffend den weiteren Farbkanal in Abhängigkeit von den erfassten Bilddaten für die Gruppe von einem oder mehreren Farbkanälen und der ermittelten Korrelation erzeugt wird.

8. Verfahren nach dem vorangehenden Anspruch, bei dem das medizinische Objekt (10) das Referenzobjekt ist oder bei dem das Referenzobjekt hinsichtlich seiner Wechselwirkung mit Licht für abzubildende medizinische Objekte (10) typisch ist.

9. Verfahren nach einem der vorangehenden Ansprüche, bei dem die Fluoreszenz von Indocyaningrün angeregt wird.

10. Verfahren nach einem der vorangehenden Ansprüche, bei dem nur eine Kamera (51, 52, 53) zum Erfassen der Bilddaten verwendet wird, wobei die Kamera einen Bildsensor (53) für mehrere Farbkanäle oder mehrere Bildsensoren für je einen Farbkanal aufweist.

11. Computer-Programm mit Programmcode zur Durchführung oder Steuerung eines Verfahrens nach einem der vorangehenden Ansprüche.

12. Vorrichtung (60) zum Steuern einer mehrfarbigen Ausgabe eines Bilds eines medizinischen Objekts (10), mit:
einem oder mehreren Bildsensoren (53) zum Erfassen von Bilddaten, für eine Gruppe von einem oder mehreren Farbkanälen;
einer Rekonstruktionseinrichtung (72), die mit einem Ausgang des einen oder der mehreren Bildsensoren (53) gekoppelt ist, zum Erzeugen von Bildinformation betreffend einen weiteren Farbkanal, der nicht zu der Gruppe von einem oder mehreren Farbkanälen zählt, in Abhängigkeit von den erfassten Bilddaten und ohne Verwendung von Bilddaten betreffend den weiteren Farbkanal;
einer Steuerung (73), die mit dem Ausgang des einen oder der mehreren Bildsensoren (53) und mit einem Ausgang der Rekonstruktionseinrichtung (72) gekoppelt ist, zum Steuern einer Bildausgabeeinrichtung (79) zur mehrfarbigen Ausgabe des Bilds in Abhängigkeit von den erfassten Bilddaten und der erzeugten Bildinformation;
einer Beleuchtungseinrichtung (31, 32) zum Beleuchten des medizinischen Objekts (10) mit Licht mit einem Beleuchtungsspektrum und mit Licht mit einem Anregungsspektrum, das von dem Beleuchtungsspektrum verschieden ist,
so dass das medizinische Objekt (10) aufgrund der Beleuchtung mit dem Anregungsspektrum Fluoreszenzlicht emittiert,
wobei der eine oder die mehreren Bildsensoren (53) ferner zum Erfassen von Bilddaten für den weiteren Farbkanal ausgebildet ist,
und das Fluoreszenzlicht über den weiteren Farbkanal erfasst wird,
wobei die Steuerung (73) ferner zum Steuern der Bildausgabeeinrichtung (79) in Abhängigkeit von den erfassten weiteren Bilddaten oder in Abhängigkeit von den erfassten Bilddaten, der erzeugten Bildinformation und den erfassten weiteren Bilddaten ausgebildet ist.

13. Vorrichtung (60) nach dem vorangehenden Anspruch, wobei die Vorrichtung zur Ausführung oder Steuerung eines Verfahrens nach einem der vorangehenden Verfahrensansprüche ausgebildet ist.

## Claims

1. Method for controlling a multi-colour output of an image of a medical object (10) for assisting medical staff, comprising the following steps:
illuminating (111) the medical object (10) with light having an illumination spectrum;
acquiring (112) image data for a group of one or more colour channels by means of an image sensor (53);
generating (115) image information in relation to a further colour channel, which is not part of the group of one or more colour channels, as a function of the acquired image data, wherein no image data relating to the further colour channel are included in the generation of the image information;
controlling (117) an image output apparatus (79) for the multi-colour output of the image as a function of the acquired image data and the generated image information;
illuminating (111) the medical object (10) with light having an excitation spectrum, which differs from the illumination spectrum;
acquiring (116) further image data for the further colour channel by means of the image sensor (53) or a further image sensor,
wherein the image output apparatus (79) is controlled as a function of the acquired further image data or as a function of the acquired image data, the generated image information and the acquired further image data,
wherein the medical object (10) emits fluorescence light due to the illumination with the excitation spectrum
and the fluorescence light is acquired by means of the further colour channel.

2. Method according to the preceding claim, wherein illuminating (111) and acquiring (112) are implemented by way of an endoscopic probe (20).

3. Method according to one of the preceding claims, wherein image information for a pixel is generated at least either as a function of the acquired image data for this pixel or as a function of acquired image data for surrounding pixels.

4. Method according to one of the preceding claims, wherein image information for a pixel is generated as a function of image data acquired at different times.

5. Method according to the preceding claim, wherein
image data for a plurality of images of the medical object (10) are acquired in succession,
the medical object (10) or part of the medical object (10) is at least either identified or tracked in the plurality of images in order to assign pixels to the medical object (10) or the part of the medical object (10),
image information for a pixel is generated as a function of the assignment of the pixel to the medical object (10) or to the part of the medical object (10).

6. Method according to one of the preceding claims, wherein a lookup table is used for generating image information.

7. Method according to one of the preceding claims, further comprising the following step:
acquiring (102) reference image data for the group of one or more colour channels and for the further colour channel when a reference object is illuminated (101) by white light or light with a different predetermined spectrum;
establishing (106) a correlation between the reference image data for the further colour channel and reference image data for the group of one or more colour channels,
wherein the image information relating to the further colour channel is generated as a function of the acquired image data for the group of one or more colour channels and of the established correlation.

8. Method according to the preceding claim, wherein the medical object (10) is the reference object or wherein the reference object is typical in respect of the interaction thereof with light for medical objects (10) to be imaged.

9. Method according to one of the preceding claims, wherein the fluorescence of indocyanine green is excited.

10. Method according to one of the preceding claims, wherein only one camera (51, 52, 53) is used for acquiring the image data, wherein the camera has an image sensor (53) for a plurality of colour channels or a plurality of image sensors for respectively one colour channel.

11. Computer program comprising program code for executing or controlling a method according to one of the preceding claims.

12. Device (60) for controlling a multi-colour output of an image of a medical object (10), comprising:
one or more image sensors (53) for acquiring image data for a group of one or more colour channels;
a reconstruction apparatus (72), which is coupled to an output of the one or more image sensors (53), for generating image information in relation to a further colour channel, which is not part of the group of one or more colour channels, as a function of the acquired image data and without using image data in relation to the further colour channel;
a control unit (73), which is coupled to the output of the one or more image sensors (53) and to one output of the reconstruction apparatus (72), for controlling an image output apparatus (79) for the multi-colour output of the image as a function of the acquired image data and the generated image information;
an illumination apparatus (31, 32) for illuminating the medical object (10) with light having an illumination spectrum and with light having an excitation spectrum, which differs from the illumination spectrum,
such that the medical object (10) emits fluorescence light due to the illumination with the excitation spectrum,
wherein the one or more image sensors (53) are furthermore embodied for acquiring image data for the further colour channel,
and the fluorescence light is acquired by way of the further colour channel,
wherein the control unit (73) is furthermore embodied to control the image output apparatus (79) as a function of the acquired further image data or as a function of the acquired image data, the generated image information and the acquired further image data.

13. Device (60) according to the preceding claim, wherein the device is embodied for performing or controlling a method according to one of the preceding method claims.

## Revendications

1. Procédé de commande d'une sortie multicolore d'une image d'un objet médical (10) pour assister un personnel médical, comprenant les étapes consistant à :
éclairer (111) l'objet médical (10) avec une lumière présentant un spectre d'éclairement ;
acquérir (112) des données d'images pour un groupe d'un ou plusieurs canaux de couleurs au moyen d'un capteur d'image (53) ;
générer (115) des informations d'images concernant un autre canal de couleur qui ne fait pas partie du groupe d'un ou plusieurs canaux de couleurs en fonction des données d'images acquises, dans lequel, lors de la génération des informations d'images, aucune donnée d'image concernant l'autre canal de couleur n'est prise en compte ;
commander (117) un dispositif de sortie d'image (79) afin qu'il délivre une sortie multicolore de l'image en fonction des données d'images acquises et des informations d'images générées ;
éclairer (111) l'objet médical (10) avec une lumière présentant un spectre d'excitation qui est différent du spectre d'éclairement ;
acquérir (116) d'autres données d'images pour l'autre canal de couleur au moyen du capteur d'image (53) ou d'un autre capteur d'image,
dans lequel le dispositif de sortie d'image (79) est commandé en fonction des autres données d'images acquises ou en fonction des données d'images acquises, des informations d'images générées et des autres données d'images acquises,
dans lequel l'objet médical (10) émet une lumière de fluorescence sur la base de l'éclairement présentant le spectre d'excitation,
et la lumière de fluorescence est acquise par l'intermédiaire de l'autre canal de couleur.

2. Procédé selon l'une quelconque des revendications précédentes, dans lequel l'éclairement (111) et l'acquisition (112) sont effectués par l'intermédiaire d'une sonde endoscôpique (20).

3. Procédé selon l'une quelconque des revendications précédentes, dans lequel des informations d'images correspondant à un point d'image sont générées au moins soit en fonction des données d'images acquises pour ce point d'image, soit en fonction de données d'images acquises pour des points d'images voisins.

4. Procédé selon l'une quelconque des revendications précédentes, dans lequel des informations d'images correspondant à un point d'image sont générées en fonction des données d'images détectées à des instants différents.

5. Procédé selon l'une quelconque des revendications précédentes, dans lequel des données d'images consécutives sont acquises sur une pluralité d'images de l'objet médical (10),
l'objet médical (10) ou une partie de l'objet médical (10) est au moins soit identifié, soit poursuivi dans la pluralité d'images afin d'associer des points d'images à l'objet médical (10) ou à la partie de l'objet médical (10),
des informations d'images correspondant à un point d'image sont générées en fonction de l'association du point d'image avec l'objet médical (10) ou avec la partie de l'objet médical (10).

6. Procédé selon l'une quelconque des revendications précédentes, dans lequel une table de consultation est utilisée pour générer des informations d'images.

7. Procédé selon l'une quelconque des revendications précédentes, comportant en outre les étapes consistant à :
acquérir (102) des données d'images de référence correspondant aux groupes d'un ou plusieurs canaux de couleurs et à l'autre canal de couleur lors de l'éclairement (101) d'un objet de référence avec de la lumière blanche ou une lumière présentant un autre spectre prédéterminé ;
déterminer (106) une corrélation entre des données d'images de référence correspondant à l'autre canal de couleur et des données d'images de référence correspondant au groupe d'un ou plusieurs canaux de couleurs,
dans lequel les informations d'images concernant l'autre canal de couleur sont générées en fonction des données d'images acquises pour le groupe d'un ou plusieurs canaux de couleurs et de la corrélation obtenue.

8. Procédé selon l'une quelconque des revendications précédentes, dans lequel l'objet médical (10) est l'objet de référence ou dans lequel l'objet de référence est typique d'objets médicaux dont les images doivent être formées (10) en ce qui concerne son interaction avec la lumière.

9. Procédé selon l'une quelconque des revendications précédentes, dans lequel la fluorescence est excitée par du vert d'indocyanine.

10. Procédé selon l'une quelconque des revendications précédentes, dans lequel on n'utilise qu'une seule caméra (51, 52, 53) pour acquérir les données d'images, dans lequel la caméra comprend un capteur d'image (53) pour une pluralité de canaux de couleurs ou une pluralité de capteurs d'images pour chaque canal de couleur.

11. Programme informatique comportant un code de programme pour mettre en oeuvre ou commander un procédé selon l'une quelconque des revendications précédentes.

12. Dispositif (60) destiné à commander une sortie multicolore d'une image d'un objet médical (10), comportant :
un ou plusieurs capteurs d'images (53) pour acquérir des données d'images pour un groupe d'un ou plusieurs canaux de couleurs ;
un dispositif de reconstruction (72) qui est couplé à une sortie desdits un ou plusieurs capteurs de couleur (53) pour générer des informations d'images concernant un autre canal de couleur qui ne fait pas partie du groupe d'un ou plusieurs canaux de couleurs, en fonction des données d'images acquises ou sans utiliser les données d'images concernant l'autre canal de couleur ;
une unité de commande (73) qui est couplée à la sortie desdits un ou plusieurs capteurs d'images (53) et à une sortie du dispositif de reconstruction (72), pour commander un dispositif de sortie d'image (79) permettant une sortie multicolore de l'image en fonction des données d'images acquises et des informations d'images générées ;
un dispositif d'éclairement (31, 32) destiné à éclairer l'objet médical (10) avec une lumière présentant un spectre d'éclairement et avec une lumière présentant un spectre d'excitation qui est différent du spectre d'éclairement,
de manière à ce que l'objet médical (10) émette une lumière de fluorescence sur la base de la lumière présentant le spectre de stimulation,
dans lequel lesdits un ou plusieurs capteurs d'images (53) sont en outre conçus pour acquérir des données d'images pour l'autre canal de couleur ;
et la lumière de fluorescence est acquise par l'intermédiaire de l'autre canal de couleur,
dans lequel l'unité de commande (73) est en outre conçue pour commander le dispositif de sortie d'image (79) en fonction des autres données d'images acquises ou en fonction des données d'images acquises, des informations d'images générées et des autres données d'images acquises.

13. Dispositif (60) selon la revendication précédente, dans lequel le dispositif est conçu pour mettre en oeuvre ou commander un procédé selon l'une quelconque des revendications précédentes.
